# EUROPÄISCHE PATENTSCHRIFT

(11) **EP 1 734 365 B1**
(45) Veröffentlichungstag und Bekanntmachung des Hinweises auf die Patenterteilung: **06.01.2010**
(21) Anmeldenummer: 05012701.8
(22) Anmeldetag: 14.06.2005
(51) Int. Cl.: G01N 33/487

(54) **Körperflüssigkeit-Analysevorrichtung mit einem Testfelder aufweisenden Testband**
Body fluid analysis device with test band having test fields
Dispositif d'analyse d'un liquide corporel, avec une bande test présentante des zones test

(43) Veröffentlichungstag der Anmeldung: 20.12.2006
(73) Patentinhaber: F. Hoffmann-La Roche AG, 4070 Basel (CH); Roche Diagniostics GMBH, 68305 Mannheim (DE)
(72) Erfinder: Sacherer, Klaus-Dieter, 67281 Kirchheim (DE)
(74) Vertreter: Pfiz, Thomas

(56) Entgegenhaltungen:
- WO-A-2004/056269
- DE-A1- 10 244 775
- DE-A1- 19 715 031

## Beschreibung

Die Erfindung betrifft eine Vorrichtung zur Analyse einer Körperflüssigkeit, insbesondere für Blutzuckertests, mit einem Testband, welches ein Trägerband und eine Vielzahl von längs über das Trägerband verteilten und gegenüber diesem erhöhten Testfeldern für den Nachweis eines Analyten in der Körperflüssigkeit umfasst, und mit einem eine Dichtung zum Durchzug des Testbands aufweisenden Behälter für das Testband, wobei die Testfelder eine beim Durchzug gegen einen Dichtungsrand anlaufende Anlaufkante besitzen.

Solche Testbänder lassen sich vor allem zur Blutzuckerbestimmung als Verbrauchskassetten in automatisch arbeitenden Handgeräten einsetzen, mit denen auch von Laien die erforderlichen Untersuchungsschritte einfach und schnell vorgenommen werden können. Anstelle herkömmlicher einzelner Teststreifen sind auf dem aufgewickelten Analyseband eine Vielzahl von mit einer geeigneten Testchemie versehenen Testfeldern fortlaufend angeordnet. Die Körperflüssigkeit wird dabei auf einem durch Bandvorlauf in eine aktive Position gebrachten Testfeld appliziert, um sodann einen Nachweis beispielsweise durch eine optische Untersuchung zu ermöglichen. Auf diese Weise lässt sich eine Vielzahl von Tests durchführen, ohne dass eine separate Handhabung und Entsorgung disposibler Teststreifen erforderlich wäre.

In der WO 2004/056269 A1 der Anmelderin sind verschiedene Dichtungskonzepte beschrieben, um den unverbrauchten Teil des Testbands in einem Behältnis gegen schädliche Umwelteinflüsse zu schützen und zugleich einen Bandtransport für die sukzessive Bereitstellung der Testfelder zu erlauben. Dabei werden auch Dichtungen mit Dichtlippen offenbart, die parallel zur Vorderkante der Testfelder verlaufen.

Ausgehend hiervon liegt der Erfindung die Aufgabe zugrunde, die im Stand der Technik aufgetretenen Nachteile zu vermeiden und eine gattungsgemäße Vorrichtung dahingehend zu verbessern, dass der Bandlauf auch hinsichtlich der Anforderungen an den Antrieb weiter optimiert wird.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die Geometrie der Testfelder und der Dichtung aufeinander anzupassen, so dass der Banddurchzug erleichtert wird. Dementsprechend wird erfindungsgemäß vorgeschlagen, dass die in Bandtransportrichtung weisende Anlaufkante der Testfelder oder/und der bezüglich der Testfelder wirksame Dichtungsrand zumindest bereichsweise gegenüber der Querrichtung des Trägerbands abgewinkelt oder abgekrümmt sind. Dadurch wird erreicht, dass Anlaufkante und Dichtungsrand einen von Null verschiedenen Winkel einschließen, so dass ein abrupter Kantenkontakt über eine größere Breite vermieden wird und die Kraftspitze beim Durchziehen des jeweiligen Testfelds durch die Dichtung erniedrigt bzw. abgeflacht wird.

Damit die Krafterhöhung allmählich über die Bandbreite erfolgt, ist es von Vorteil, wenn die Testfelder eine gegen den Dichtungsrand weisende, durch die Anlaufkante begrenzte Spitze aufweisen.

Vorteilhafterweise verläuft die Anlaufkante der Testfelder über deren Breite von 90° abweichend schräg zur Bandlängsrichtung. Entsprechende Vorteile werden auch erreicht, wenn der mit dem Testband in Eingriff stehende Dichtungsrand über die Bandbreite schräg zur Bandlängsrichtung verläuft.

Um das Auftreten von Kraftspitzen zu vermeiden, sollten der Dichtungsrand und die Anlaufkante einen Winkel von mindestens 5° einschließen.

Eine weitere Verbesserung sieht vor, dass die Dichtung für einen sanften Bandeinlauf profiliert, vorzugsweise in Höhenrichtung des Testbands abgerundet ist.

Besonders bevorzugt wird das erfindungsgemäße Dichtungskonzept bei einem durch eine Kassette gebildeten Behälter eingesetzt, wobei die Dichtung eine Vorratskammer der Kassette für das Testband im Auslassbereich abdichtet.

Für eine gute Dichtwirkung ist es vorteilhaft, wenn die Dichtung einen gegen die die Testfelder tragende Breitseite des Trägerbands anpressbaren flexiblen Dichtungsbereich aufweist.

Herstellungstechnisch ist es günstig, wenn die Testfelder als vorgefertigte Flachmaterialteile auf das Testband aufgebracht, vorzugsweise aufgeklebt sind.

Ein Erfindungsaspekt ist auch darin zu sehen, dass die Anlaufkante der Testfelder beim Durchzug durch die Dichtung abschnittsweise den Dichtungsrand passiert.

Im Folgenden wird die Erfindung anhand der in der Zeichnung schematisch dargestellten Ausführungsbeispiele näher erläutert. Es zeigen
- Fig. 1: eine Testbandkassette für Blutzuckertests mit einem abgedichteten Banddurchlass in einer perspektivischen, teilweise aufgebrochenen Darstellung;

- Fig. 2: ein Testband mit schräg gestellten Testfeldern in einer ausschnittsweisen Draufsicht;
- Fig. 3: eine weitere Ausführungsform in einer Fig. 2 entsprechenden Darstellung; und
- Fig. 4: ein Kraftdiagramm für den Banddurchzug durch eine herkömmliche Dichtung nach dem Stand der Technik.

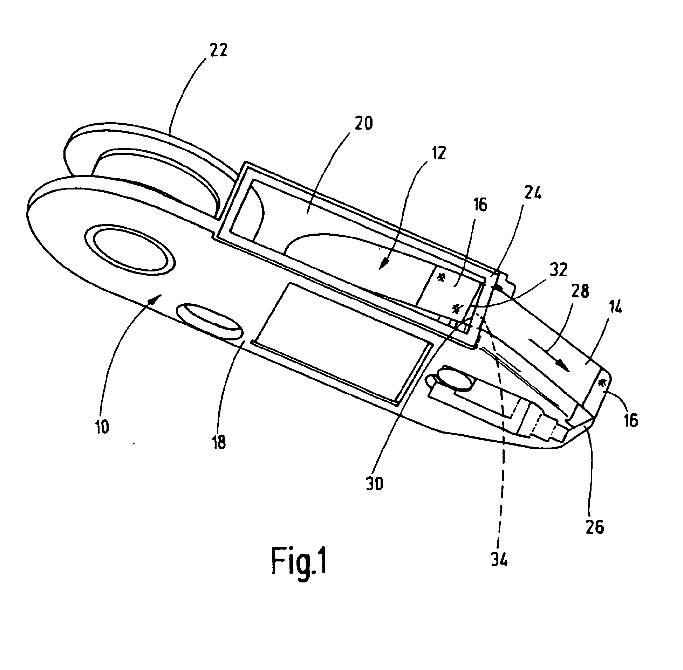

Die in Fig. 1 gezeigte Testbandkassette 10 enthält ein Testband 12, welches ein Trägerband 14 und eine Vielzahl von in Bandlängsrichtung im Abstand voneinander auf dem Trägerband angebrachten Testfeldern 16 für den Blutzuckernachweis in einer Blutprobe umfasst. Die Kassette 10 weist ein Behältnis bzw. Aufnahmegehäuse 18 mit einem Vorratsraum 20 für unverbrauchtes Testband zweckmäßig in Form eines Bandwickels und mit einer Abzugspule 22 für verbrauchtes Testband auf. Eine Dichtung 24 dichtet den Vorratsraum 20 statisch gegen eine nicht gezeigte Abdeckung nach außen ab, während ein Dichtungsschlitz den Durchzug des Testbandes 12 erlaubt, so dass unverbrauchte Testfelder 16 an einer Umlenkspitze 26 mit Probenflüssigkeit bzw. Blut beaufschlagt werden können.

Die Testfelder 16 sind als vorgefertigte, etikettenartige Zuschnittteile auf das Trägerband 14 aufgeklebt und demzufolge gegenüber diesem erhöht. Sie weisen somit eine in Bandzugrichtung (Pfeil 28) gegen einen Dichtungsrand 30 der Dichtung 24 anlaufende Anlaufkante 32 auf. Bei dem in Fig. 1 gezeigten Beispiel verläuft die Anlaufkante 32 der Testfelder 16 über die Breite des Trägerbandes 14 orthogonal zur Bandlängsrichtung, während der einlassseitige Dichtungsrand 30 unter einem spitzen Winkel schräg dazu verläuft, so dass ein sanfter Banddurchzug möglich ist. Hierfür sollte der von dem Dichtungsrand 30 und der Anlaufkante 32 angeschlossene Winkel mindestens 5° betragen.

Zweckmäßig ist der Dichtungsrand 30 für einen sanften Bandeinlauf zusätzlich profiliert, insbesondere von der Bandbreitseite weg nach oben abgerundet. Der wirksame Dichtungsrand wird hierbei durch diejenige Dichtlinie definiert, die mit dem Trägerband 14 bzw. dem Testfeldaufbau 16 in Eingriff kommt. Im Gegensatz zu einer berührungsfreien Spaltdichtung weist die Dichtung 24 einen gegen das Trägerband 14 anpressbaren flexiblen Dichtungsbereich 34 für eine schleifende Flächendichtung auf.

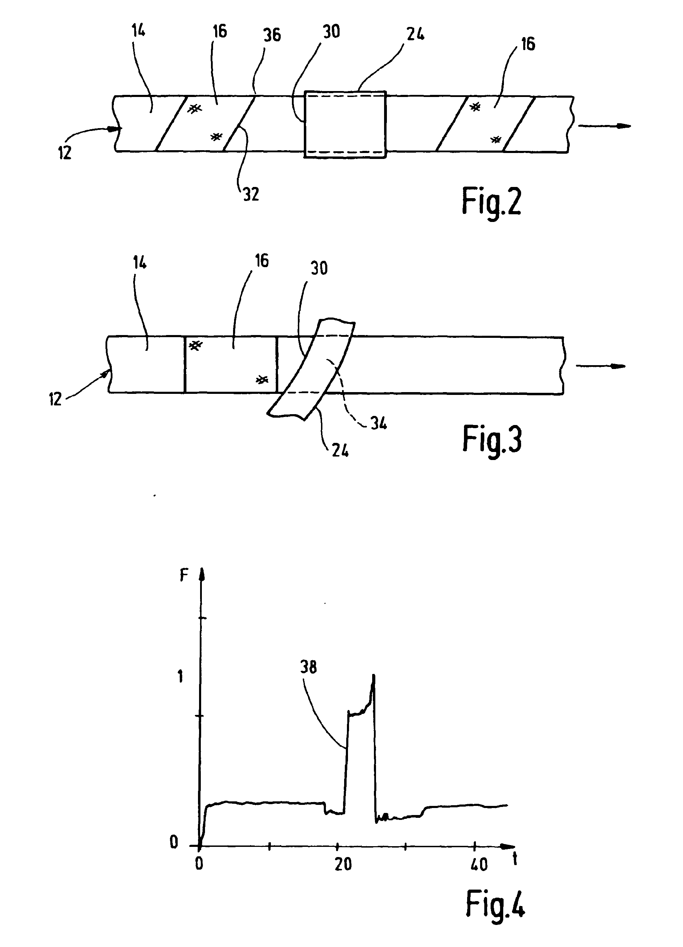

Bei der in Fig. 2 gezeigten Ausführungsform wird durch Schrägstellung der Testfelder 16 auf dem Trägerband 14 das Auftreten von Kraftspitzen beim Durchzug durch die Dichtung 24 vermieden. Die Anlaufkante 32 verläuft also von 90° abweichend schräg zur Bandlängskante. Auf diese Weise besitzen die Testfelder 16 eine gegen den orthogonal zur Bandlängsrichtung verlaufenden Dichtungsrand 30 weisende Spitze 36, welche zunächst den Dichtungsrand 30 passiert, so dass die Bandantriebskraft nur allmählich werden muss, um den Durchzugwiderstand zu überwinden.

In der Ausführungsform nach Fig. 3 sind wie in Fig. 1 rechteckförmige Testfelder 16 auf dem Trägerband 14 aufgebracht, während der Dichtungsrand 30 der Dichtung 24 abgekrümmt in Bandquerrichtung verläuft. Auch bei dieser Maßnahme wird ein sanfter Eintritt der erhöhten Testfelder 16 in den Dichtungsbereich 34 gewährleistet.

Während somit durch die erfindungsgemäße Schrägstellung der Testfelder 16 bzw. Dichtung 24 Kraftspitzen vermieden werden, zeigt Fig. 4 ein Kraftdiagramm für den Banddurchzug bei einer Anordnung nach dem Stand der Technik, bei der Dichtungsrand 30 und Anlaufkante 32 parallel zueinander verlaufen. Hier tritt beim Durchlauf eines Testfelds durch die Dichtung ein sprungartiger Kraftanstieg 38 auf, was insgesamt zu einem unruhigen Bandlauf führt und einen starken Bandantrieb erfordert.

## Patentansprüche

1. Vorrichtung zur Analyse einer Körperflüssigkeit, insbesondere für Blutzuckertests, mit einem Testband (12), welches ein Trägerband (14) und eine Vielzahl von längs über das Trägerband (14) verteilten und gegenüber diesem erhöhten Testfeldern (16) für den Nachweis eines Analyten in der Körperflüssigkeit umfasst, und mit einem eine Dichtung (24) zum Durchzug des Testbands (12) aufweisenden Behälter (18) für das Testband (12), wobei die Testfelder (16) eine beim Durchzug gegen einen Dichtungsrand (30) anlaufende Anlaufkante (32) besitzen, **dadurch gekennzeichnet, dass** die Anlaufkante (32) oder/und der Dichtungsrand (30) zumindest bereichsweise gegenüber der Bandquerrichtung abgewinkelt oder abgekrümmt sind.

2. Vorrichtung nach Anspruch 1, **dadurch gekennzeichnet, dass** die Testfelder (16) eine gegen den Dichtungsrand (30) weisende, durch die Anlaufkante (32) begrenzte Spitze (36) aufweisen.

3. Vorrichtung nach Anspruch 1 oder 2, **dadurch gekennzeichnet, dass** die Anlaufkante (32) der Testfelder (16) über deren Breite von 90° abweichend schräg zur Bandlängsrichtung verläuft.

4. Vorrichtung nach Anspruch 1 oder 2, **dadurch gekennzeichnet, dass** der mit dem Testband (12) in Eingriff stehende Dichtungsrand (30) über die Bandbreite schräg zur Bandlängsrichtung verläuft.

5. Vorrichtung nach einem der Ansprüche 1 bis 4, **dadurch gekennzeichnet, dass** der Dichtungsrand (30) und die Anlaufkante (32) einen Winkel von mindestens 5° einschließen.

6. Vorrichtung nach einem der Ansprüche 1 bis 5, **dadurch gekennzeichnet, dass** die Dichtung (30) für einen sanften Bandeinlauf profiliert, vorzugsweise in Höhenrichtung des Testbands (12) abgerundet ist.

7. Vorrichtung nach einem der Ansprüche 1 bis 6, **dadurch gekennzeichnet, dass** der Behälter (18) durch eine Kassette (10) gebildet ist, wobei die Dichtung (24) eine Vorratskammer (20) der Kassette (10) für das Testband (12) im Auslassbereich abdichtet.

8. Vorrichtung nach einem der Ansprüche 1 bis 7, **dadurch gekennzeichnet, dass** die Dichtung (24) einen gegen die die Testfelder (16) tragende Breitseite des Trägerbands (14) anpressbaren flexiblen Dichtungsbereich (34) aufweist.

9. Vorrichtung nach einem der Ansprüche 1 bis 8, **dadurch gekennzeichnet, dass** die Testfelder (16) als vorgefertigte Flachmaterialteile auf das Testband (12) aufgebracht, vorzugsweise aufgeklebt sind.

## Claims

1. Device for analysing a body fluid in particular for blood sugar tests comprising a test tape (12) which consists of a carrier tape (14) and a plurality of test fields (16) for the detection of an analyte in the body fluid that are distributed along the carrier tape (14) and are raised above the tape and comprising a container (18) for the test tape (12) having a seal (24) for passage of the test tape (12), wherein the test fields (16) have a leading edge (32) which runs against a sealing edge (30) when the tape is pulled through, **characterized in that** the leading edge (32) or/and the sealing edge (30) at least in parts are angled or curved relative to the transverse direction of the tape.

2. Device according to claim 1, **characterized in that** the test fields (16) have a tip (36) delimited by the leading edge (32) that points towards the sealing edge (30).

3. Device according to claim 1 or 2, **characterized in that** the leading edge (32) of the test fields (16) is diagonal to the longitudinal direction of the tape at an angle that differs from 90° over their width.

4. Device according to claim 1 or 2, **characterized in that** the sealing edge (30) interacting with the test tape (12) extends diagonally to the longitudinal direction of the tape over the tape width.

5. Device according to one of the claims 1 to 4, **characterized in that** the sealing edge (30) and the leading edge (32) enclose an angle of at least 5°.

6. Device according to one of the claims 1 to 5, **characterized in that** the seal (30) is profiled for a gentle tape entry and is preferably rounded in the vertical direction of the test tape (12).

7. Device according to one of the claims 1 to 6, **characterized in that** the container (18) is formed by a cassette (10) where the seal (24) seals a storage chamber (20) of the cassette (10) for the test tape (12) in the outlet area.

8. Device according to one of the claims 1 to 7, **characterized in that** the seal (24) has a flexible sealing area (34) that can be pressed against the broad side of the carrier tape (14) that carries the test fields (16).

9. Device according to one of the claims 1 to 8, **characterized in that** the test fields (16) are applied to the test tape (12) as prefabricated flat material components and are preferably glued on.

## Revendications

1. Dispositif d'analyse d'un liquide corporel, en particulier pour tests de glycémie, avec une bande de test (12), laquelle comprend une bande support (14) et une pluralité de zones de test (16) réparties le long de la bande support (14) et réalisées en relief par rapport à celle-ci pour la détection d'un analyte dans le liquide corporel, et avec un récipient (18) pour la bande de test (12), lequel est pourvu d'un joint (24) pour le passage de la bande de test (12), les zones de test (16) possédant un bord d'attaque (32) butant contre un bord de joint (30) au moment du passage, **caractérisé en ce que** le bord d'attaque (32) et/ou le bord de joint (30) sont au moins partiellement repliés ou recourbés par rapport au sens transversal de bande.

2. Dispositif selon la revendication 1, **caractérisé en ce que** les zones de test (16) présentent une pointe (36) dirigée contre le bord de joint (30) et délimitée par le bord d'attaque (32).

3. Dispositif selon la revendication 1 ou 2, **caractérisé en ce que** le bord d'attaque (32) des zones de test (16) s'étend sur la largeur de celles-ci obliquement selon un angle différent de 90° par rapport au sens longitudinal de bande.

4. Dispositif selon la revendication 1 ou 2, **caractérisé en ce que** le bord de joint (30) en prise avec la bande de test (12) s'étend sur la largeur de bande obliquement par rapport au sens longitudinal de bande.

5. Dispositif selon l'une des revendications 1 à 4, **caractérisé en ce que** le bord de joint (30) et le bord d'attaque (32) enferment un angle au moins égal à 5°.

6. Dispositif selon l'une des revendications 1 à 5, **caractérisé en ce que** le joint (30), en vue d'une introduction douce de la bande, est profilé, de préférence arrondi vers le haut de la bande de test (12).

7. Dispositif selon l'une des revendications 1 à 6, **caractérisé en ce que** le récipient (18) est formé par une cartouche (10), le joint (24) assurant l'étanchéité d'un compartiment de réserve (20) de la cartouche (10) pour la bande de test (12) dans la zone de sortie.

8. Dispositif selon l'une des revendications 1 à 7, **caractérisé en ce que** le joint (24) présente une zone d'étanchéité (34) flexible, apte à se plaquer contre le côté large de la bande support (14) portant les zones de test (16).

9. Dispositif selon l'une des revendications 1 à 8, **caractérisé en ce que** les zones de test (16) sont appliquées, de préférence collées sur la bande de test (12) sous forme de pièces préfabriquées en matériau plat.
